# EUROPÄISCHE PATENTSCHRIFT

(11) **EP 2 770 956 B1**
(45) Veröffentlichungstag und Bekanntmachung des Hinweises auf die Patenterteilung: **06.12.2017**
(21) Anmeldenummer: 12778716.6
(22) Anmeldetag: 24.10.2012
(51) Int. Cl.: A61F 2/64, A61F 2/68, A61F 2/60, A61F 2/70

(54) **AKTIVE KNIEPROTHESE MIT KEGELSTIRNRADGETRIEBE**
ACTIVE KNEE PROSTHESIS COMPRISING BEVEL GEAR
PROTHÈSE ACTIVE DU GENOU MUNIE D'UN ENGRENAGE CYLINDRIQUE CONIQUE

(30) Priorität: 24.10.2011 DE 102011116751
(43) Veröffentlichungstag der Anmeldung: 03.09.2014
(73) Patentinhaber: Fraunhofer-Gesellschaft zur Förderung der angewandten Forschung, 80636 München (DE)
(72) Erfinder: BUDAKER, Bernhard, 71336 Waiblingen (DE); HOVY, Lars, 81373 München (DE); FEILER, Thomas, 75177 Pforzheim (DE)
(74) Vertreter: Pfenning, Meinig & Partner mbB
(86) Internationale Anmeldenummer: PCT/EP2012/071080
(87) Internationale Veröffentlichungsnummer: WO 2013/060742

(56) Entgegenhaltungen:
- WO-A1-03/017876
- WO-A1-2007/063266
- US-A1- 2006 249 315

## Beschreibung

Die Erfindung betrifft eine Knieprothese, welche die erforderlichen Drehmoment- und Winkelgeschwindigkeiten entlang der gesamten Antriebskette ausschließlich rotatorisch bewirkt. Hierzu wird das Drehmoment von einem antreibenden Motor auf das Kniegelenk über ein Kegelstirnradgetriebe übertragen.

Aus dem Stand der Technik sind verschiedene aktive Knie-Prothesen bekannt. So beschreibt die WO 2005/051248 A1 eine Knieprothese, welche die Bewegung mit Hilfe eines Hebelarmmechanismus umsetzt. Dabei wird die Energie in der Antriebskette von einem linearen Aktor (Kombination Motor und Hebelarmgetriebe) aufgebracht. Das System ist vor allem durch die Anwendung des Hebelarms sehr groß und schwer. Der Hebelarm benötigt zwangsläufig eine minimale Größe, um die geforderten Drehmomente am Knie aufbringen zu können.

Die US 2006/0249315 A1 stellt den nächstliegenden Stand der Technik dar und beschreibt verschiedene Gelenke für künstliche Gliedmaßen.
Auf dem Gebiet der passiven Knieprothesen und der aktiv gesteuerten Knieprothesen (die nicht aktiv angetrieben werden) gibt es eine Vielzahl von Systemen, welche allerdings keine aktiv unterstützte Fortbewegung ermöglichen. Durch aktiv gesteuerte Dämpfersysteme kann jedoch während der Bewegung eine gewisse Standsicherheit und adaptive Dämpfung des Kniegelenks ermöglicht werden.
Aufgabe der vorliegenden Erfindung ist es, eine Knieprothese anzugeben, welche eine leichte Erlernbarkeit des Gangs mit Prothesen für Amputierte ermöglicht und gleichzeitig die zur Verfügung stehende Energie energetisch effizient umsetzt. Vorteilhaft soll die Knieprothese auch für ältere Menschen verwendbar sein und preislich mit passiven Knieprothesen vergleichbar sein.
Die Aufgabe wird gelöst durch die Knieprothese nach Anspruch 1. Die abhängigen Ansprüche geben vorteilhafte Weiterbildungen der erfindungsgemäßen Knieprothese an.
Erfindungsgemäß wird eine aktiv angetriebene Knieprothese angegeben, welche die Bewegung vorteilhaft in rotatorischer Form umsetzt. Die gesamte Antriebskette kann hierbei rotatorisch realisiert werden, ohne dass die Verwendung von Hebelmechanismen notwendig wäre.
Die erfindungsgemäße Knieprothese weist einen Oberschenkelteil und einen Unterschenkelteil auf. Die Prothese kann implantierbar ausgebildet sein; dabei wird der Oberschenkelteil der Prothese mit dem bestehen gebliebenen Teil des amputierten Oberschenkelknochens verbunden. Bevorzugt ist die Prothese jedoch als Exo-Prothese ausgebildet, welche einen amputierten Unterschenkel und ein amputiertes Knie ersetzt. Die Prothese wird mit dem Oberschenkelteil an einem Prothesenschaft über einen sogenannten Pyramidenadapter angebracht. Vorteilhaft weist der Oberschenkelteil an einer der Knieachse abgewandten Seite einen Adapter auf, vorzugsweise eine genormte Adapterplatte, mit welcher die erfindungsgemäße Knieprothese mit einem Schaftsystem in Richtung des Oberschenkels verbindbar ist. Darüberhinaus weist vorzugsweise der Unterschenkelteil an seinem dem Kniegelenk abgewandten Ende einen Adapter, vorzugsweise eine genormte Adapterplatte auf, über die die Knieprothese mit einem Fuß-System verbindbar ist.

Erfindungsgemäß sind Oberschenkelteil und Unterschenkelteil gegeneinander um eine Gelenkachse drehbar, die der Knieachse entspricht bzw. die Knieachse ist.

Zum Antreiben des Kniegelenks weist die Knieprothese einen Motor auf, der einen um eine Drehachse drehbaren Rotor aufweist. Die erfindungsgemäße Knieprothese weist außerdem ein Kegelradgetriebe auf, über welches ein von dem Motor um die Drehachse bewirktes Drehmoment in ein Drehmoment um die Gelenkachse übertragbar ist. Durch dieses Drehmoment um die Gelenkachse ist dann der Oberschenkelteil gegenüber dem Unterschenkelteil um die Gelenkachse drehbar. Ein vom Rotor des Motors bereitgestelltes Drehmoment wird also in ein Drehmoment zur Bewegung des Oberschenkelteils gegenüber dem Unterschenkelteil übertragen. Generell kann jener Teil der Knieprothese, über den der Oberschenkelteil gegen den Unterschenkelteil bewegbar ist, als Kniegelenk angesehen werden und wirkt in der Prothese als solches.

Es ist bevorzugt, wenn der Motor an oder in der Knieprothese so angeordnet ist, dass die Drehachse senkrecht zur Gelenkachse steht. Insbesondere kann der Motor im Oberschenkelteil oder im Unterschenkelteil angeordnet sein und dabei besonders bevorzugt so angeordnet sein, dass die Drehachse des Rotors parallel zu einer Längsrichtung des Oberschenkelteils bzw. des Unterschenkelteils liegt. Auf diese Weise kann die Knieprothese besonders platzsparend und einfach realisiert werden.
Es ist bevorzugt, wenn zwischen dem Rotor des Motors und dem Kegelradgetriebe (also dessen durch den Motor angetriebenen Kegelrad) ein Getriebe, vorzugsweise ein Planetengetriebe angeordnet ist, mit welchem ein vom Motor bewirktes Drehmoment in ein auf das vom Motor angetriebene Kegelrad des Kegelradgetriebes wirkendes Drehmoment übertragbar ist. Ein solches Getriebe kann vorteilhaft direkt auf den Motor aufgeflanscht sein. Das Übersetzungsverhältnis in diesem Getriebe kann insbesondere bei einem Planetengetriebe variabel ausgelegt werden. Dies kann dann auch abhängig vom Einsatzgebiet der Prothese variiert werden. So kann beispielsweise bei einem höheren Körpergewicht und/oder einer geringeren körperlichen Aktivität des Patienten ein höheres Übersetzungsverhältnis gewählt werden.
Das genannte Kegelradgetriebe als Teil eines Kegelstirnradgetriebes dient zur kinematischen Übersetzung der vom Motor bzw. vom Motor und ggf. dem Getriebe aufgebrachten Leistung. Das Kegelradgetriebe oder Kegelstirnradgetriebe nimmt auf der einen Seite die Kräfte und Drehmomente, die um die Gelenkachse wirken, auf und ermöglicht auf der anderen Seite eine entsprechende Erzeugung einer Bewegung an der Gelenkachse. Vorteilhafterweise können vom Motor nach Übersetzung über das Kegelstirnradgetriebe Drehmomente erzeugbar, die eine vollständige Bewegung des Kniegelenkes ohne Unterstützung durch weitere Drehmomente ermöglichen.

In einer vorteilhaften Ausgestaltung der Erfindung weist das Kegelradgetriebe ein erstes und ein zweites Kegelrad auf, wobei eines der Kegelräder vom Motor, ggf. über ein weiteres Getriebe, angetrieben wird und das andere Kegelrad als nicht vom Motor angetriebenes Kegelrad die Bewegung um die Gelenkachse antreibt, vorzugsweise über weitere Getriebeteile. Vorteilhafterweise stehen die Drehachsen der Kegelräder des Kegelradgetriebes in einem Winkel von 90° zueinander. Dies ist insbesondere von Vorteil, wenn die Drehachse des Motors parallel zur Längsrichtung des Unterschenkelteils oder des Oberschenkelteils liegt, da normalerweise die Gelenkachse senkrecht zu dieser Längsrichtung steht. Ein derartiges Kegelradgetriebe erfordert also nur eine Änderung der Ausrichtung der Drehachse.

Bevorzugterweise liegt die Drehachse des vom Motor angetriebenen Kegelrades mit der Drehachse des Rotors des Motors auf einer gemeinsamen Geraden und vorteilhaft auch auf einer gemeinsamen Geraden mit dem zwischen Kegelstirnradgetriebe und Motor angeordneten Getriebe, sofern ein solches vorgesehen ist.

Jenes Kegelrad des Kegelradgetriebes, welches nicht das vom Motor angetriebene Kegelrad ist, kann direkt oder über zumindest ein zwischengeordnetes Zahnrad den Teil des Kniegelenks, also Oberschenkelteil oder Unterschenkelteil, antreiben, der nicht den Motor aufweist. Hierzu kann vorteilhaft das nicht vom Motor angetriebene Kegelrad oder ein direkt oder mittelbar durch dieses Kegelrad angetriebenes Zahnrad in ein mit dem entsprechenden Teil der Prothesen fest verbundenen Zahnrad oder einem gezahnten Bereich eingreifen und diesen antreiben. Das zumindest eine weitere Zahnrad bzw. der gezahnte Bereich, vorteilhafterweise mit weiteren Zahnrädern, ist Teil eines Stirnradgetriebes oder bildet ein solches, so dass das Kegelradgetriebe und das Stirnradgetriebe zusammen ein Kegelstirnradgetriebe bilden.

Die erfindungsgemäße Knieprothese weist vorteilhaft eine Drehmomentstütze auf, mit der eine Rotation eines Stators des Motors gegenüber der Drehachse verhinderbar ist. Diese Drehmomentstütze stützt also die Bewegung des Motors ab, so dass der Rotor ein Drehmoment um die Drehachse auf das Kniegelenk ausüben kann. Vorzugsweise ist die Drehmomentstütze fest mit einem das Kegelrad-oder Kegelstirnradgetriebe haltenden Teil der Knieprothese verbunden.

Vorteilhaft kann die Drehmomentstütze Teil einer Gehäuseeinheit sein, die den Unterschenkelteil oder den Oberschenkelteil der Knieprothese bildet bzw. Teil dieses Teils ist. Vorteilhafterweise wird dieses Gehäuse als Leichtbaueinheit realisiert und insbesondere kann es aus Kunststoff geeignet hergestellt sein. Vorteilhafterweise ist das Gehäuse bezüglich des Kraftflusses optimiert. Das Gehäuse kann dann die Antriebseinheit bzw. den Motor abstützen und eine Ableitung der von der Antriebseinheit bzw. dem Motor aufgebrachten Kraft ermöglichen. In einer vorteilhaften Ausgestaltung kann das Gehäuse aus drei Wänden zusammengesetzt sein, die in Längsrichtung des Oberschenkelteils oder Unterschenkelteils länger sind als in hierzu zu senkrechter Richtung breit. Die drei Teile können an ihren Rändern so miteinander verbunden sein, dass sie in einer Ebene senkrecht zur genannten Längsrichtung einen rechteckigen Querschnitt des Gehäuses realisieren. Der Querschnitt hat hierbei also eine rechteckige U-Form. Die zwei einander gegenüberliegenden Flächen des Gehäuses und bevorzugt alle drei Flächen des Gehäuses können sich in Richtung von der Gelenkachse weg verjüngen. In einem dem Kniegelenk abgewandten Endbereich können sich dann vorteilhaft die beiden gegenüberliegenden Flächen so verbreitern, dass hier eine genormte Adapterplatte anbringbar ist, über welche die Prothese mit einem Fußsystem oder einem Schaftsystem verbindbar ist.

Sofern es auf ein Gewicht der Knieprothese nicht ankommt oder die Knieprothese zu Testzwecken realisiert wird, kann sie vorteilhaft in einem Dynamikprüfstand realisiert werden, der dann den Oberschenkelteil oder den Unterschenkelteil bildet oder Teil dieses Teils ist. Ein solcher Dynamikprüfstand kann den Motor und die Getriebe aufnehmen. Er hat vorteilhaft in einer Ebene senkrecht zur Längsrichtung einen quadratischen oder rechteckigen Querschnitt, z.B. Käfigform. Er kann zusammengesetzt sein aus einer im Wesentlichen rechteckigen Platte, die parallel zur genannten Längsrichtung verläuft und in dieser Richtung länger ist als in einer hierzu senkrechten Richtung breit. An jenen dieser Platte abgewandten Ecken des rechteckigen Querschnitts kann der Dynamikprüfstand zylinderförmige Säulen aufweisen, die mit ihrer Zylinderachse parallel zur Längsrichtung verlaufen. Wiederum kann eine Adapterplatte zur Verbindung der Knieprothese mit einem Schaftsystem oder einem Fußsystem an jenem der Gelenkachse abgewandten Ende des Dynamikprüfstands angeordnet sein. Der Dynamikprüfstand ermöglicht für die Entwicklung von Antriebssystemen in Knieprothesen eine modulare Plattform. Modular bedeutet in diesem Fall, dass verschiedene Antriebssysteme, also Kombinationen von Motoren- und Getriebe-Einheiten im Dynamikprüfstand getestet werden können. Der Dynamikprüfstand kann in der Höhe variabel einstellbar sein und kann verschiedene Unterschenkelhöhen und somit verschiedene Unterschenkelvarianten simulieren. Er kann für den Motor variable Befestigungspunkte aufweisen.
Sowohl das optimierte Gehäuse als auch der Dynamikprüfstand können so ausgestaltet sein, dass sie das Kegelstirnradgetriebe sowie alle durch dieses angetriebenen Getriebeteile wie Zahnräder trägt, die nicht fest mit dem jeweils anderen Teil aus Oberschenkelteil oder Unterschenkelteil verbunden sind. Vorteilhaft trägt dann das Gehäuse bzw. der Dynamikprüfstand auch den Motor.
Der Motor, d.h. die Antriebseinheit der Knieprothese, ist vorteilhaft ein Elektromotor und besonders bevorzugt ein elektronisch kommutierter Motor bzw. ein bürstenloser Gleichstrommotor.
Jenes nicht vom Motor angetriebene Kegelrad des Kegelradgetriebes treibt den nicht den Motor aufweisenden Teil der Knieprothese über ein Stirnradgetriebe an. Das Drehmoment wird also von diesem Kegelrad auf den entsprechenden Teil der Prothese vorteilhaft über n Stufen übertragen, so dass zwischen dem durch den Motor angetriebenen Kegelrad und dem entsprechenden Teil der Prothese ein bestimmtes Übersetzungsverhältnis i realisiert wird. Bevorzugterweise ist dabei n ≥ 1, besonders bevorzugt ≥ 3 und/oder ≤ 10, vorzugsweise ≤ 5. Das Übersetzungsverhältnis i ist vorteilhaft ≥ 3, vorzugsweise ≥ 5 und/oder ≤ 10, besonders bevorzugt ≤ 8.
Die Leistung am Systemeingang (Drehmoment und Drehzahl, motorseitig) wird also über n Stufen (beispielsweise drei Stufen über drei Achsen, wobei eine davon die Knieachse darstellt) in die Leistung am Systemausgang umgesetzt. Hierbei wird dann ein Übersetzungsverhältnis i (beispielsweise i=5) realisiert. Die letzte Getriebestufe ist hierbei mit dem Anschluss des Schaftsystems (Schnittstelle zum Schaftsystem) fest verbunden.
In analoger Weise ist es bevorzugt, wenn das Planetengetriebe - zusätzlich oder alternativ zum Übersetzungsverhältnis des Kegelstirnradgetriebes - ein Übersetzungsverhältnis zwischen 9:1 und 25:1 aufweist. Vorteilhaft kann die Knieprothese zumindest einen Energiespeicher, z.B. einen Akku, aufweisen, welcher den Motor mit Energie versorgt.
Vorteilhafterweise ist durch den Motor nach Übersetzung durch das Kegelstirnradgetriebe ein Drehmoment um die Gelenkachse bzw. Knieachse von größer oder gleich 40 Nm, vorzugsweise größer oder gleich 60 Nm, vorzugsweise größer oder gleich 80 Nm, bewirkbar und/oder eine Winkelgeschwindigkeit von größer oder gleich 200 Grad pro Sekunde, vorzugsweise größer oder gleich 300 Grad pro Sekunde, vorzugsweise 400 Grad pro Sekunde, bewirkbar.

Die erfindungsgemäße Knieprothese ermöglicht es, den kompletten Gangzyklus "nachzufahren". Das Drehmoment und die Winkelgeschwindigkeitskurven können daher ohne passive Unterstützung, also ohne hydraulische, pneumatische oder mechanische Dämpferelemente, nachgefahren werden. Insbesondere kann das Kniegelenk ausschließlich vom Motor angetrieben werden.

Das Knieprothesensystem kann rein elektromechanisch angetrieben realisiert werden und die Bewegungsumsetzung kann in rein rotatorischer Form erfolgen. Hierdurch können Verluste vermieden werden, welche durch die Umwandlung von linearen in rotatorische Bewegungsformen und wieder zurück zwangsläufig auftreten. Das Antriebssystem stellt eine besonders kostengünstige Lösung dar, welche sich durch die Einfachheit des Aufbaus auszeichnet, insbesondere wenn das System rein elektromechanisch ausgeführt wird.

Das Gehäuse kann als reines Kunststoff-Spritzguss-System aufgebaut werden und ermöglicht dann eine besonders kostengünstige Herstellung sowie eine hohe Gewichtsreduktion.

Die erfindungsgemäße Knieprothese ermöglicht eine kompakte Bauform, die sich durch den Einsatz von rein rotatorischen Energie- und Leistungsübertragungselementen auszeichnet. Motor und Getriebeeinheit können hierbei verschieden zueinander angeordnet sein, jedoch erfolgt vorteilhaft an der Knieachse eine Übersetzung in die entsprechenden Drehmomente. Dies wird durch entsprechend ausgelegte Getriebeübersetzungsverhältnisse realisiert. Die kinematische Umsetzung am Knie ermöglicht schließlich das Aufbringen der entsprechenden Drehmoment-Drehzahlkennlinien. Die kinematische Übersetzung kann z.B. entweder durch eine weitere Getriebeeinheit oder aber auch durch eine spezielle mechanische Konstruktion realisiert werden.

Im Folgenden soll die Erfindung anhand einiger Figuren beispielhaft erläutert werden. Gleiche Bezugszeichen entsprechen gleichen oder analogen Merkmalen.

Die in den Figuren gezeigten Merkmale können auch unter den verschiedenen Figuren kombiniert werden und unabhängig von der konkreten Ausgestaltung realisiert sein.

Es zeigt
- Figur: 1 eine Antriebseinheit einer erfindungsgemäßen Knieprothese,
- Figur 2: eine in einem Dynamikprüfstand eingebaute Antriebseinheit der erfindungsgemäßen Knieprothese,
- Figur 3: eine seitliche Ansicht der im Dynamikprüfstand eingebauten Antriebseinheit, wie sie in Figur 2 gezeigt ist,
- Figur 4: ein einen Oberschenkelteil antreibendes Getriebe mit einem Kegelstirnradgetriebe,
- Figur 5: das in Figur 4 gezeigte Getriebe mit relativen Drehrichtungen in einer beispielhaften Drehung,
- Figur 6: den in Figur 2 und 3 gezeigten Dynamikprüfstand ohne Antriebseinheit und
- Figur 7: einen optimierten Kunststoffgehäuseaufbau zur Aufnahme der Antriebseinheit als Teil einer Knieprothese.

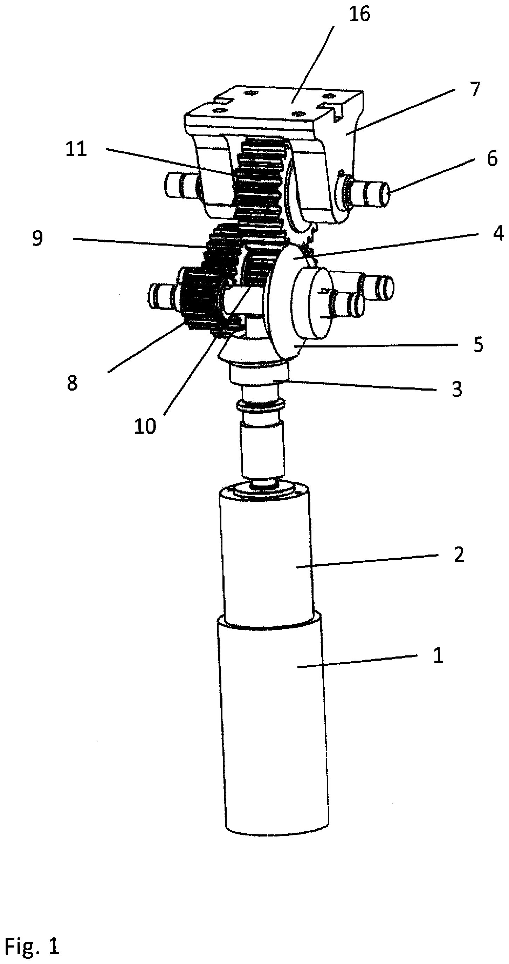

Figur 1 zeigt beispielhaft eine Antriebseinheit, wie sie in der erfindungsgemäßen Knieprothese realisiert sein kann. Die Antriebseinheit weist einen Motor 1 auf, auf den ein Getriebe, beispielsweise ein Planetengetriebe 2, aufgeflanscht ist. Das Getriebe 2 treibt ein Kegelrad 3 eines Kegelradgetriebes 5 an. Drehachsen eines Rotors des Motors 1, des Getriebes 2 und des Kegelrades 3 liegen auf einer gemeinsamen Geraden, die sich im eingebauten Zustand des Antriebsystems in einer erfindungsgemäßen Knieprothese in Richtung parallel zu einer Längsrichtung des entsprechenden Teils aus Unterschenkelteil oder Oberschenkelteil der Prothese erstrecken.

In den folgenden Beispielen soll die Erfindung unter der Annahme beschrieben werden, dass der Motor im Unterschenkelteil der Knieprothese angeordnet ist. Analog kann der Motor jedoch auch im Oberschenkelteil angeordnet sein.

Das Kegelrad 3 treibt ein weiteres Kegelrad 4 des Kegelradgetriebes 5 an, dessen Drehachse senkrecht zur Drehachse des Kegelrades 3 steht. Die Drehachse des Kegelrades 4 liegt parallel zur Gelenkachse 6. Über das Getriebe 2, das optional ist, das Kegelrad 3 und das Kegelrad 4 wird der Unterschenkelteil gegenüber einem Oberschenkelteil 7 um die Gelenkachse 6 bewegt, die der Knieachse 6 entspricht.

Im gezeigten Beispiel treibt das Kegelrad 4 ein Zahnrad 8 an, dessen Drehachse mit der Drehachse des Kegelrads 4 zusammenfällt. Das Zahnrad 8 greift in ein größeres Zahnrad 9 ein, dessen Drehachse parallel zur Drehachse des Zahnrades 8 liegt. Auf der Drehachse des größeren Zahnrades 9 ist ein weiteres Zahnrad 10 angeordnet, das eine geringere Zahl an Zähnen und einen geringeren Durchmesser als das Zahnrad 9 aufweist. Dieses weitere Zahnrad 10 greift in ein größeres Zahnrad 11 ein, welches mit dem Oberschenkelteil 7 fest verbunden ist und dessen Drehachse 6 mit der Gelenkachse 6 zusammenfällt. Das Zahnrad 11 könnte auch als gezahnter Bereich des Oberschenkelteils 7 ausgestaltet sein. Die Zahnräder 8, 9, 10 und 11 bilden ein Stirnradgetriebe und zusammen mit dem Kegelradgetriebe 5 ein Kegelstirnradgetriebe. Das Drehmoment vom nicht durch den Motor 1 angetriebenen Kegelrad 4 auf den Oberschenkel 7 kann hier also über n Stufen des Kegelstirnradgetriebes übertragen werden, so dass zwischen dem durch den Motor 1 angetriebenen Kegelrad 3 und dem Oberschenkelteil 7 oder dem Unterschenkelteil ein Übersetzungsverhältnis von i realisiert wird, wobei vorzugsweise n ≥ 1, besonders bevorzugt n ≥ 3 und/oder n ≤ 10, vorzugsweise n ≤ 5 und/oder i ≥ 3, vorzugsweise i ≤ 5 und/oder ≤ 10, vorzugsweise i ≤ 8 ist.

Die erfindungsgemäße Knieprothese kann eine Drehmomentstütze aufweisen, die eine Rotation eines Stators des Motors 1 gegenüber der Drehachse und/oder dem Rotor verhindert, wobei vorzugsweise die Drehmomentstütze fest mit einem das Kegelstirnradgetriebe haltenden Teil der Knieprothese verbunden ist.

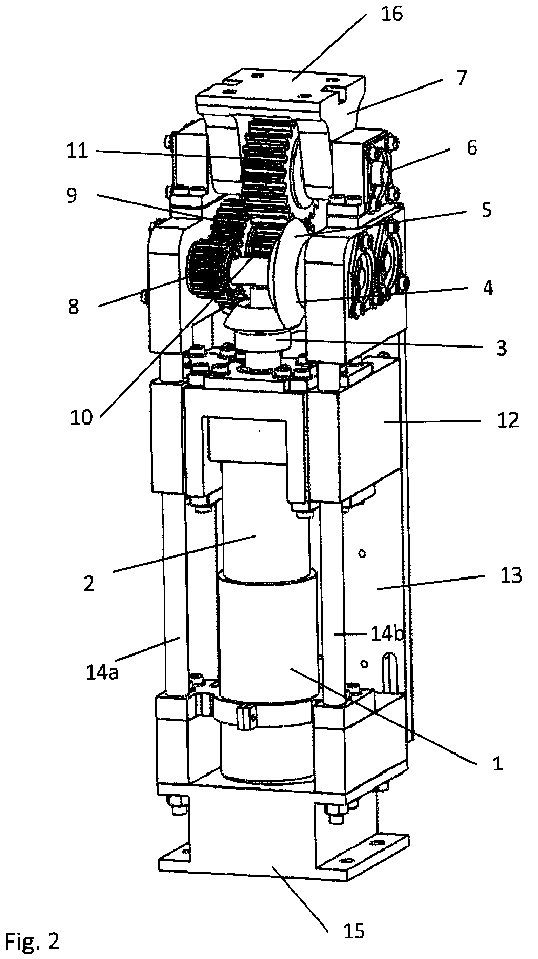

Figur 2 zeigt das in Figur 1 gezeigte Antriebssystem eingebaut in einen Dynamikprüfstand 12. Der Dynamikprüfstand 12 wird hierbei als Teil des Unterschenkelteils der Knieprothese angesehen. Der Dynamikprüfstand 12 trägt den Motor, das Getriebe 2, das Kegelstirnradgetriebe 5 sowie die weiteren Zahnräder 8, 9, 10. Um die Gelenkachse 6 bzw. Knieachse 6 ist der Oberschenkelteil 7 gegenüber dem Unterschenkelteil und dem Dynamikprüfstand 12 drehbar. Im gezeigten Beispiel weist der Dynamikprüfstand 12 eine rechteckige Platte 13 auf, die länglich ausgestaltet ist und sich mit ihrer Längsrichtung parallel zur Drehachse des Motors 1 erstreckt. Parallel zu den Kanten dieser Platte 13 weist der Dynamikprüfstand zwei zylinderförmige Säulen 14a und 14b auf, die sich mit ihrer Zylinderachse parallel zur Drehachse des Motors 1 erstrecken. An jenem der Gelenkachse 6 abgewandten Ende des Dynamikprüfstands 12 ist ein Adapter 15 angeordnet, der beispielsweise eine normierte Adapterplatte 15 sein kann, über welche die Knieprothese an ein Fuß-System angeschlossen werden kann. Die Adapterplatte 15 ist also im Normalfall dem Boden zugewandt. Der Oberschenkelteil 7 kann entsprechend eine Adapterplatte 16 aufweisen, über die die Knieprothese an ein Schaftsystem angeschlossen werden kann und die in Richtung Oberschenkel weist. Der Dynamikprüfstand kann durch Verlängerung oder Verkürzung der Säulen 14a und 14b sowie der entsprechenden Anpassung an der Platte 13 in der Länge variiert werden. Die Winkelstellung und Achspositionen der Antriebseinheit im Dynamikprüfstand von Motor und Getriebeachse(n) können ebenfalls variiert werden.

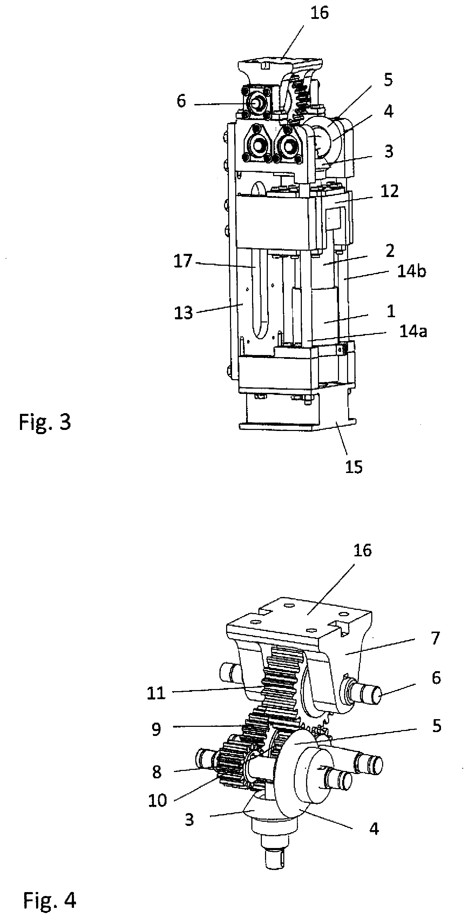

Figur 3 zeigt die Antriebseinheit in dem Dynamikprüfstand 12 entsprechend Abbildung 2. Während Figur 2 eine perspektivische Ansicht einer Seite der Vorrichtung ist, zeigt Figur 3 eine perspektivische Ansicht auf die gegenüberliegende Seite. Die zu Figur 2 gemachten Ausführungen gelten hier entsprechend. In Figur 3 ist die Anordnung der Zahnräder und Kegelräder gut zu erkennen. Das Kegelrad 4 des Kegelstirnradgetriebes liegt mit seiner Achse parallel zur Achse der Zahnräder 9 und 10 und auf gleicher Höhe in Richtung einer Längsrichtung, die in Richtung der Drehachse des Motors 1 verläuft. Die Drehachse 6, welche die Gelenkachse bzw. Knieachse darstellt, liegt oberhalb dieser Achsen.

Im gezeigten Beispiel ist der Adapter 15 an den Dynamikprüfstand 12 am von der Achse 6 abgewandten Ende angeschraubt.

Figur 4 zeigt nur den Übertragungsteil des Antriebssystems, wie es in Figur 1 gezeigt ist. Wie in Figur 1 beschrieben, greift das Kegelrad 3 mit dem Kegelrad 4 des Kegelradgetriebes 5 ineinander, welches seinerseits das Zahnrad 8 antreibt. Das Zahnrad 8 greift mit dem größeren Zahnrad 9 ineinander, das mit dem kleineren Zahnrad 10 auf einer gemeinsamen Achse angeordnet ist und dieses dreht. Das kleinere Zahnrad 10 greift dann in das Zahnrad 11 ein, das mit dem Oberschenkelteil 7 fest verbunden ist und gemeinsam mit diesem um die Gelenkachse 6 bzw. Knieachse 6 dreht.

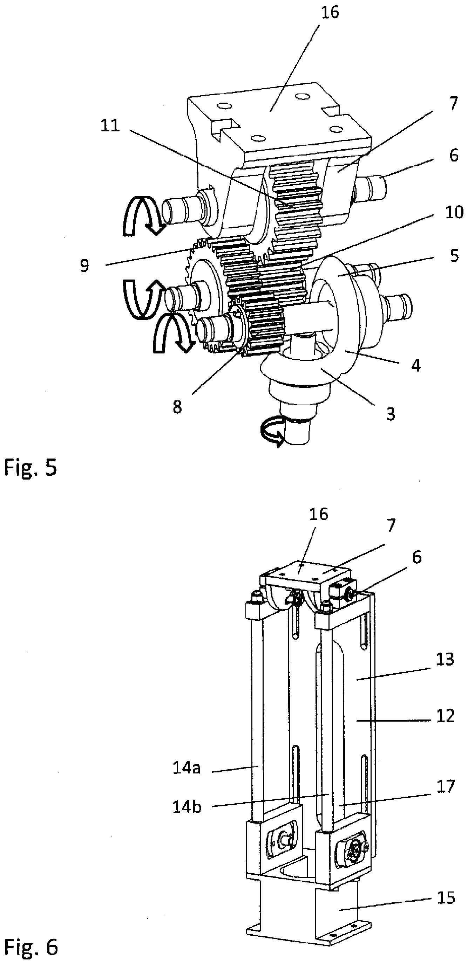

Figur 5 zeigt das in Figur 4 gezeigte Getriebe aus einer anderen perspektivischen Ansicht. Dabei sind beispielhaft die Drehrichtungen der Zahnräder eingezeichnet. Selbstverständlich können alle Drehrichtungen auch umgekehrt wirken, wenn der Motor 1 in entgegengesetzter Richtung dreht. Im gezeigten Beispiel dreht der Motor 1 das Kegelrad 3 aus Sicht des Motors betrachtet rechts herum. Dadurch drehen sich das Kegelrad 4 und das kleine Zahnrad 8 in Richtung vom kleinen Zahnrad 8 zum Kegelrad 4 betrachtet im Uhrzeigersinn. Da das kleine Zahnrad 8 mit dem größeren Zahnrad 9 ineinandergreift, dreht sich dieses in gleicher Richtung betrachtet entgegen den Uhrzeigersinn. Entsprechend dreht sich auch das auf der gleichen Achse angeordnete Zahnrad 10 entgegen den Uhrzeigersinn. Das Zahnrad 10 greift mit dem Zahnrad 11 ein, das um die Gelenkachse 6 dreht und den Oberschenkelteil 7 bewegt. Diese drehen sich also in gleicher Richtung betrachtet im Uhrzeigersinn. Dreht der Motor 1 das Kegelrad 3 in entgegengesetzter Richtung, werden alle genannten Drehrichtungen umgekehrt und der Oberschenkelteil dreht in die entgegengesetzte Richtung.

Figur 6 zeigt beispielhaft einen Unterschenkelteil ohne Antriebseinheit, der auch als Dynamikprüfstand fungieren kann. Der Aufbau entspricht im Wesentlichen dem in Figur 2 gezeigten Aufbau. Das Gehäuse 12 weist hierbei eine Rückenplatte 13 auf, die sich in einer Längsrichtung parallel zur Drehachse des Motors 1 erstreckt. Parallel zu dieser Drehachse sind außerdem zwei zylinderförmige Stützen 14a und 14b angeordnet. Die Stützen 14a und 14b bilden zusammen mit den parallel zur Längsrichtung verlaufenden Kanten der Rückplatte 13 die Kanten eines Quaders. Das Gehäuse 12 trägt am oberen Ende die Gelenkachse 6 und den Oberschenkelteil 7 mit der Adapterplatte 16. Am unteren Teil ist der Adapter 15 angeschraubt. Die Rückplatte 13 weist zur Gewichtsreduktion eine längliche Öffnung 17 auf, die sich mit ihrer Längsrichtung parallel zur Drehachse 1 des Motors und zur Längsrichtung der Rückplatte 13 erstreckt.

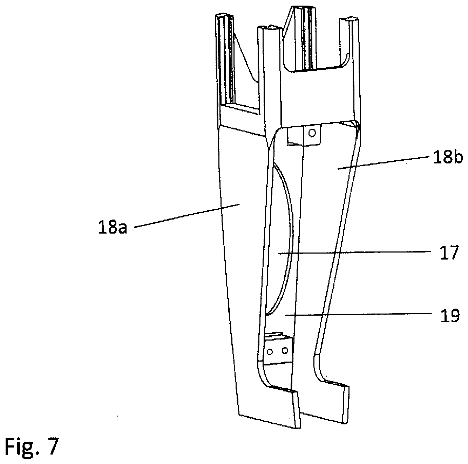

Figur 7 zeigt ein gewichts- und kraftübertragungsoptimiertes Gehäuse zum Einsatz im Unterschenkelteil der erfindungsgemäßen Knieprothese. Die Antriebseinheit und die Gelenke sind hierbei nicht gezeigt. Das Gehäuse weist drei Seitenplatte 18a, 18b und 19 auf, die sich entlang einer Längsrichtung des Gehäuses erstrecken, die bei eingebautem Motor 1 mit der Drehachse des Motors zusammenfällt. In einer Ebene senkrecht zur dieser Drehachse grenzen die Platten 18a, 18b und 19 einen rechteckigen Querschnitt ein. Am oberen Teil des in Figur 7 gezeigten Gehäuses kann die Zahnradmechanik und der Oberschenkelteil mit der Gelenkachse angeordnet werden und am unteren Teil kann ein Adapter zur Aufnahme eines Fußsystems angeordnet sein. Die Platten 18a, 18b und 19 verjüngen sich in Richtung des unteren Endes, wobei sich jedoch die Seitenplatten 18a und 18b am unteren Ende in einem Endbereich so verbreitern, dass hier der Adapter 15 anordenbar ist. Die Rückplatte 19 weist eine Öffnung 17 auf, die der Gewichtsreduktion dient.

Die Knieprothese kann ein Gehäuse aufweisen, dass Teil des Unterschenkelteils oder des Oberschenkelteils ist und welches als Drehmomentstütze für den Stator des Motors 1 wirkt, und welches das Kegelstirnradgetriebe 5 trägt.

In den gezeigten Beispielen kann der Motor beispielsweise ein Elektromotor, vorzugsweise ein elektronisch kommutierter Motor oder ein bürstenloser Gleichstrommotor sein.

Im folgenden soll ein Beispiel für eine mögliche Bemessung der Knieprothese angegeben werden. Es kann das Kegelstirnradgetriebe eine Gesamtübersetzung von i=5:1 haben. Die Kegelräder können als Zyklo-Palloid-Spiralverzahnung mit speziell gehärteten Flanken ausgebildet sein, wodurch eine besonders hohe Kraftübertragung möglich wird. Eine Übersetzung des Kegelradgetriebes kann z.B. 1,5:1 betragen. Ein mittlerer Durchmesser des Antriebsrades kann z.B. 32 mm bei 16 Zähnen betragen und ein mittlerer Durchmesser des getriebenen Rades 48 mm bei 24 Zähnen. Das treibende Kegelrad kann beispielsweise ein Dauerdrehmoment von M=11,9 Nm aufnehmen, das getriebene Kegelrad z.B. 17,9 Nm. Das Getriebe kann insgesamt, also um die Gelenkachse 6 bzw. Knieachse 6, mit einem Dauerdrehmoment von z.B. 65 bis 120 Nm belastet werden.

## Patentansprüche

1. Knieprothese mit
einem Oberschenkelteil (7) und einem Unterschenkelteil, die gegeneinander um eine Gelenkachse (6) drehbar sind,
einem Motor (1), der einen um eine Drehachse drehbaren Rotor aufweist,
**gekennzeichnet durch** ein Kegelstirnradgetriebe, das aus einem Kegelradgetriebe (5) und einem Stirnradgetriebe gebildet wird und über das ein von dem Motor (1) um die Drehachse bewirktes Drehmoment in ein Drehmoment um die Gelenkachse (6) übertragbar ist, mit welchem der Oberschenkelteil (7) gegenüber dem Unterschenkelteil um die Gelenkachse (6) drehbar ist.

2. Knieprothese nach dem vorhergehenden Anspruch, wobei die Drehachse senkrecht zur Gelenkachse (6) steht.

3. Knieprothese nach einem der vorhergehenden Ansprüche, wobei der Motor (1) im Oberschenkelteil (7) oder im Unterschenkelteil angeordnet ist und vorzugsweise die Drehachse parallel zu einer Längsrichtung des Oberschenkelteils oder des Unterschenkelteils liegt.

4. Knieprothese nach einem der vorhergehenden Ansprüche mit einem zwischen dem Rotor und dem Kegelstirnradgetriebe angeordneten weiteren Getriebe (2), mit dem ein vom Motor bewirktes Drehmoment in ein auf das Kegelstirnradgetriebe wirkendes Drehmoment übertragbar ist, wobei das weitere Getriebe (2) vorzugsweise ein Planetengetriebe (2) ist.

5. Knieprothese nach einem der vorhergehenden Ansprüche, wobei das Kegelstirnradgetriebe ein erstes (3) und ein zweites (4) Kegelrad aufweist, deren Drehachsen vorzugsweise in einem Winkel von 90° zueinander stehen.

6. Knieprothese nach dem vorhergehenden Anspruch, wobei eine Drehachse eines der Kegelräder (3) mit der Drehachse des Rotors auf einer gemeinsamen Geraden liegt und, auf der vorzugsweise auch eine Drehachse des weiteren Getriebes liegt.

7. Knieprothese nach einem der Ansprüche 5 oder 6, wobei jenes Kegelrad (4), welches nicht das vom Rotor angetriebene Kegelrad ist, direkt oder über zumindest ein Zahnrad ein Zahnrad (8) antreibt, welches mit einem nicht den Motor (1) erfassenden Teil aus Oberschenkelteil (7) und Unterschenkelteil festverbundenen gezahnten Bereich oder Zahnrad (9) ineinandergreift.

8. Knieprothese nach einem der vorhergehenden Ansprüche mit einer Drehmomentstütze, die eine Rotation eines Stators des Motors (1) gegenüber der Drehachse und/oder dem Rotor verhindert, wobei vorzugsweise die Drehmomentstütze fest mit einem das Kegelstirnradgetriebe haltenden Teil der Knieprothese verbunden ist.

9. Knieprothese nach einem der vorhergehenden Ansprüche mit einem Gehäuse (12), das Teil des Unterschenkelteiles oder des Oberschenkelteils (7) ist, und welches als Drehmomentstütze für den Stator des Motors (1) wirkt und welches das Kegelstirnradgetriebe trägt, wobei vorzugsweise das Gehäuse (12) Kunststoff aufweist oder daraus besteht.

10. Knieprothese nach dem vorhergehenden Anspruch, wobei das Gehäuse (12) variable Befestigungspunkte für den Motor (1) und/oder eine variable Länge in Richtung senkrecht zur Gelenkachse (6) aufweist und/oder einen Käfigaufbau aufweist, so dass es als Dynamikprüfstand verwendbar ist.

11. Knieprothese nach einem der vorhergehenden Ansprüche, wobei der Motor (1) ein Elektromotor, vorzugsweise ein elektronisch kommutierter Motor oder ein bürstenloser Gleichstrommotor ist.

12. Knieprothese nach einem der Ansprüche 5-11 wobei das Drehmoment vom nicht durch den Motor (1) angetriebenen Kegelrad (4) auf den Oberschenkel über n Stufen eines Getriebes übertragen wird, so dass zwischen dem durch den Motor (1) angetriebenen Kegelrad (3) und dem Oberschenkelteil oder dem Unterschenkelteil ein Übersetzungsverhältnis von i realisiert wird, wobei vorzugsweise n ≥ 1, besonders bevorzugt n ≥ 3 und/oder n ≤ 10, vorzugsweise n ≤ 5 und/oder i ≥ 3, vorzugsweise i ≤ 5 und/oder ≤ 10, vorzugsweise i ≤ 8 ist.

13. Knieprothese nach einem der vorhergehenden Ansprüche, wobei das vom Motor (1) um die Drehachse erzeugte Drehmoment auf die Gelenkachse ausschließlich über Rotationen oder ausschließlich rotatorisch übertragen wird.

14. Knieprothese nach einem der vorhergehenden Ansprüche, wobei das Kniegelenk um die Gelenkachse (6) ausschließlich vom Motor (1) angetrieben wird.

15. Knieprothese nach einem der vorhergehenden Ansprüche, wobei durch den Motor (1) nach Übersetzung durch die Getriebe ein Drehmoment um die Gelenkachse von größer oder gleich 40 Nm, vorzugsweise größer oder gleich 60 Nm, vorzugsweise größer oder gleich 80 Nm, vorzugsweise größer oder gleich 120 Nm bewirkbar ist und/oder eine Winkelgeschwindigkeit von größer oder gleich 200 Grad pro Sekunde, vorzugsweise größer oder gleich 300 Grad pro Sekunde, vorzugsweise 400 Grad pro Sekunde, bewirkbar ist.

16. Knieprothese nach einem der Ansprüche 5-15, wobei die Kegelräder (3, 4) des Kegelstirnradgetriebes eine Zyklo-Palloid-Spiralverzahnung aufweisen, vorzugsweise mit gehärteten Flanken.

17. Knieprothese nach einem der Ansprüche 5-16, wobei die Kegelräder (3, 4) eine Übersetzung von 1,5:1 bewirken und/oder ein vom Motor angetriebenes Kegelrad (3) einen mittleren Durchmesser von 32 mm und ein getriebenes Kegelrad (4) einen mittleren Durchmesser von 48 mm hat und/oder dass das angetriebene Kegelrad 16 Zähne aufweist und das getriebene Kegelrad 24 Zähne aufweist.

18. Knieprothese nach einem der vorhergehenden Ansprüche, wobei das Antriebsrad (3) des Kegelstirnradgetriebes eingerichtet ist, ein Dauerdrehmoment von größer oder gleich 8 Nm, vorzugsweise größer oder gleich 10 Nm, vorzugsweise größer oder gleich 11 Nm aufzunehmen und/oder das getriebene Rad des Kegelstirnradgetriebes eingerichtet ist, ein Dauerdrehmoment von größer oder gleich 13 Nm, vorzugsweise größer oder gleich 15 Nm, vorzugsweise größer oder gleich 17 Nm aufzunehmen.

19. Knieprothese nach einem der vorhergehenenden Ansprüche, wobei die Knieprothese eingerichtet ist, mit einem Dauerdrehmoment um die Gelenkachse (6) von größer oder gleich 60 Nm, vorzugsweise größer oder gleich 90 Nm, vorzugsweise größer oder gleich 120 Nm belastet zu werden.

## Claims

1. A knee prosthesis, with
an upper leg part (7) and a lower leg part which are rotatable against each other about a joint axis (6),
a motor (1) which has a rotor which is rotatable about a rotation axis,
**characterised by** a bevel spur gear mechanism which is formed from a bevel gear mechanism (5) and a spur gear mechanism and via which a torque brought about about the rotation axis by the motor (1) can be transmitted into a torque about the joint axis (6), with which the upper leg part (7) is rotatable about the joint axis (6) relative to the lower leg part.

2. A knee prosthesis according to the preceding claim, wherein the rotation axis is perpendicular to the joint axis (6).

3. A knee prosthesis according to one of the preceding claims, wherein the motor (1) is arranged in the upper leg part (7) or in the lower leg part and preferably the rotation axis lies parallel to a longitudinal direction of the upper leg part or of the lower leg part.

4. A knee prosthesis according to one of the preceding claims, with a further gear mechanism (2) arranged between the rotor and the bevel spur gear mechanism with which a torque brought about by the motor can be transmitted into a torque acting on the bevel spur gear mechanism, the further gear mechanism (2) preferably being a planetary gear mechanism (2).

5. A knee prosthesis according to one of the preceding claims, wherein the bevel spur gear mechanism has a first (3) and a second (4) bevel gear, the axes of rotation of which are preferably at an angle of 90° to each other.

6. A knee prosthesis according to the preceding claim, wherein a rotation axis of one of the bevel gears (3) lies with the rotation axis of the rotor on a common straight line and on which preferably also a rotation axis of the further gear mechanism lies.

7. A knee prosthesis according to one of Claims 5 or 6, wherein the bevel gear (4) which is not the bevel gear which is driven by the rotor, directly or via at least one gear wheel drives a gear wheel (8) which engages in each other with a part out of upper leg part (7) and lower leg part, which does not include the motor (1),, securely connected toothed region or gear wheel (9).

8. A knee prosthesis according to one of the preceding claims, with a torque support which prevents rotation of a stator of the motor (1) relative to the rotation axis and/or the rotor, with preferably the torque support being connected securely to a part, holding the bevel spur gear mechanism, of the knee prosthesis.

9. A knee prosthesis according to one of the preceding claims, with a housing (12) which is part of the lower leg part or of the upper leg part (7), and which acts as a torque support for the stator of the motor (1) and which bears the bevel spur gear mechanism, with preferably the housing (12) having or consisting of plastics material.

10. A knee prosthesis according to the preceding claim, wherein the housing (12) has variable fixing points for the motor (1) and/or a variable length in the direction perpendicular to the joint axis (6) and/or has a cage structure, so that it can be used as a dynamic test stand.

11. A knee prosthesis according to one of the preceding claims, wherein the motor (1) is an electric motor, preferably an electronically commutated motor or a brushless DC motor.

12. A knee prosthesis according to one of Claims 5 - 11, wherein the torque is transmitted from the bevel gear (4) not driven by the motor (1) to the upper leg via n stages of a gear mechanism, so that a gear ratio of i is realised between the bevel gear (3) which is driven by the motor (1) and the upper leg part or the lower leg part, wherein preferably n ≥ 1, particularly preferably n ≥ 3 and/or n ≤ 10, preferably n ≤ 5 and/or i ≥ 3, preferably i ≤ 5 and/or ≤ 10, preferably i ≤ 8.

13. A knee prosthesis according to one of the preceding claims, wherein the torque generated by the motor (1) about the rotation axis is transmitted to the joint axis exclusively via rotations or exclusively in rotary manner.

14. A knee prosthesis according to one of the preceding claims, wherein the knee joint is driven about the joint axis (6) exclusively by the motor (1).

15. A knee prosthesis according to one of the preceding claims, wherein a torque about the joint axis of greater than or equal to 40 Nm, preferably greater than or equal to 60 Nm, preferably greater than or equal to 80 Nm, preferably greater than or equal to 120 Nm, can be brought about and/or an angular velocity of greater than or equal to 200 degrees per second, preferably greater than or equal to 300 degrees per second, preferably 400 degrees per second, can be brought about by the motor (1) after stepping-up by the gear mechanism.

16. A knee prosthesis according to one of Claims 5 - 15, wherein the bevel gears (3, 4) of the bevel spur gear mechanism have cyclo-palloid spiral toothing, preferably with hardened flanks.

17. A knee prosthesis according to one of Claims 5 - 16, wherein the bevel gears (3, 4) bring about a gear ratio of 1.5:1 and/or a bevel gear (3) which is driven by the motor has an average diameter of 32 mm and a moved bevel gear (4) has an average diameter of 48 mm, and/or that the driven bevel gear has 16 teeth and the moved bevel gear has 24 teeth.

18. A knee prosthesis according to one of the preceding claims, wherein the driving gear (3) of the bevel spur gear mechanism is set up to absorb a permanent torque of greater than or equal to 8 Nm, preferably greater than or equal to 10 Nm, preferably greater than or equal to 11 Nm, and/or the moved wheel of the bevel spur gear mechanism is set up to absorb a permanent torque of greater than or equal to 13 Nm, preferably greater than or equal to 15 Nm, preferably greater than or equal to 17 Nm.

19. A knee prosthesis according to one of the preceding claims, wherein the knee prosthesis is set up to be loaded with a permanent torque about the joint axis (6) of greater than or equal to 60 Nm, preferably greater than or equal to 90 Nm, preferably greater than or equal to 120 Nm.

## Revendications

1. Prothèse du genou, avec
une partie de cuisse (7) et une partie de jambe qui peuvent tourner l'une par rapport à l'autre autour d'un axe d'articulation (6),
un moteur (1), qui présente un rotor pouvant tourner autour d'un axe de rotation,
**caractérisée par** un engrenage droit à pignons coniques qui est formé d'un engrenage à pignons coniques et d'un engrenage droit et par le biais duquel un couple provoqué par le moteur (1) autour de l'axe de rotation peut être transposé en un couple autour de l'axe d'articulation (6) avec lequel la partie de cuisse (7) peut tourner par rapport à la partie de jambe autour de l'axe d'articulation (6).

2. Prothèse du genou selon la revendication précédente, l'axe de rotation étant perpendiculaire à l'axe d'articulation (6).

3. Prothèse du genou selon l'une des revendications précédentes, le moteur (1) étant disposé dans la partie de cuisse (7) ou dans la partie de jambe, et l'axe de rotation étant de préférence parallèle à une direction longitudinale de la partie de cuisse ou de la partie de jambe.

4. Prothèse du genou selon l'une des revendications précédentes, avec un autre engrenage (2), disposé entre le rotor et l'engrenage droit à pignons coniques, avec lequel un couple provoqué par le moteur peut être transposé en un couple agissant sur l'engrenage droit à pignons coniques, l'autre engrenage (2) étant de préférence un engrenage planétaire.

5. Prothèse du genou selon l'une des revendications précédentes, l'engrenage droit à pignons coniques présentant un premier (3) pignon conique et un deuxième (4) pignon conique dont les axes de rotation forment de préférence entre eux un angle de 90°.

6. Prothèse du genou selon la revendication précédente, un axe de rotation d'un des pignons coniques (3) étant situé sur une droite commune avec l'axe de rotation du rotor sur laquelle est de préférence situé également un axe de rotation de l'autre engrenage.

7. Prothèse du genou selon l'une des revendications 5 ou 6, le pignon conique (4) qui n'est pas le pignon conique entraîné par le rotor entraînant, directement ou par le biais d'au moins une roue dentée, une roue dentée (8) qui engrène avec une zone dentée ou une roue dentée (9) raccordée de façon fixe à une partie n'englobant pas le moteur (1) composée de la partie de cuisse (7) et de la partie de jambe.

8. Prothèse du genou selon l'une des revendications précédentes, avec un support de couple qui empêche une rotation d'un stator du moteur (1) par rapport à l'axe de rotation et/ou au rotor, le support de couple étant de préférence raccordé de façon fixe à une partie de la prothèse du genou qui retient l'engrenage droit à pignons coniques.

9. Prothèse du genou selon l'une des revendications précédentes, avec un boîtier (12) qui fait partie de la partie de jambe ou de la partie de cuisse (7) et qui agit en tant que support de couple pour le stator du moteur (1) et qui porte l'engrenage droit à pignons coniques, le boîtier (12) comportant de préférence de la matière plastique ou étant composé de matière plastique.

10. Prothèse du genou selon la revendication précédente, le boîtier (12) présentant des points de fixation variables pour le moteur (1) et/ou une longueur variable dans une direction perpendiculaire à l'axe d'articulation (6) et/ou une structure en cage de telle sorte qu'il peut être utilisé en tant que banc d'essai dynamique.

11. Prothèse du genou selon l'une des revendications précédentes, le moteur (1) étant un moteur électrique, de préférence un moteur à commutation électronique ou un moteur à courant continu sans balais.

12. Prothèse du genou selon l'une des revendications 5 à 11, le couple du pignon conique (4) non entraîné par le moteur (1) étant transmis à la cuisse par le biais de n étages d'un engrenage de telle sorte qu'un rapport de transmission de i est réalisé entre le pignon conique (3) entraîné par le moteur (1) et la partie de cuisse ou la partie de jambe, de préférence n ≥ 1, de façon particulièrement préférée n ≥ 3 et/ou n ≤ 10, de préférence n ≤ 5 et/ou i ≥ 3, de préférence i ≤ 5 et/ou ≤ 10, de préférence i ≤ 8.

13. Prothèse du genou selon l'une des revendications précédentes, le couple produit par le moteur (1) autour de l'axe de rotation étant transmis à l'axe d'articulation exclusivement par le biais de rotations ou exclusivement en rotation.

14. Prothèse du genou selon l'une des revendications précédentes, l'articulation du genou étant entraînée autour de l'axe d'articulation (6) exclusivement par le moteur (1).

15. Prothèse du genou selon l'une des revendications précédentes, dans laquelle, un couple d'articulation supérieur ou égal à 40 Nm, de préférence supérieur ou égal à 60 Nm, de préférence supérieur ou égal à 80 Nm, de préférence supérieur ou égal à 120 Nm, peut être provoqué autour de l'axe par le moteur (1) après la transmission par les engrenages, et/ou une vitesse angulaire supérieure ou égale à 200 degrés par seconde, de préférence supérieure ou égale à 300 degrés par seconde, de préférence supérieure ou égale à 400 degrés par seconde, peut être provoquée.

16. Prothèse du genou selon l'une des revendications 5 à 15, les pignons coniques (3, 4) de l'engrenage droit à pignons coniques présentant une denture hélicoïdale cyclopalloïde, de préférence avec des flancs durcis.

17. Prothèse du genou selon l'une des revendications 5-16, les pignons coniques (3, 4) provoquant une transmission de 1,5:1, et/ou un pignon conique (3) entraîné par le moteur ayant un diamètre de 32 mm, et un pignon conique (4) mené ayant un diamètre moyen de 48 mm et/ou le pignon conique entraîné présentant 16 dents et le pignon conique mené présentant 24 dents.

18. Prothèse du genou selon l'une des revendications précédentes, le pignon d'entraînement (3) de l'engrenage droit à pignons coniques étant agencé pour recevoir un couple permanent supérieur ou égal à 8 Nm, de préférence supérieur ou égal à 10 Nm, de préférence supérieur ou égal à 11 Nm, et/ou le pignon mené de l'engrenage droit à pignons coniques étant agencé pour recevoir un couple permanent supérieur ou égal à 13 Nm, de préférence supérieur ou égal à 15 Nm, de préférence supérieur ou égal à 17 Nm.

19. Prothèse du genou selon l'une des revendications précédentes, la prothèse du genou étant agencée pour être soumise à un couple permanent autour de l'axe d'articulation (6) supérieur ou égal à 60 Nm, de préférence supérieur ou égal à 90 Nm, de préférence supérieur ou égal à 120 Nm.
